# EUROPEAN PATENT SPECIFICATION

(11) **EP 1 390 757 B1**
(45) Date of publication and mention of the grant of the patent: **08.10.2008**
(21) Application number: 02706405.4
(22) Date of filing: 20.02.2002
(51) Int. Cl.: G01N 33/68, C12Q 1/68, G01N 33/564

(54) **DETERMINING CARTILAGE DEGENERATION AND AGING USING BONE MORPHOGENIC PROTEINS**
BESTIMMUNG VON KNORPEL-DEGENERATION UND -ALTERUNG UNTER VERWENDUNG VON KNOCHENMORPHOGENESEPROTEINEN
DETERMINATION DE LA DEGENERESCENCE DE CARTILAGE ET DU VIELLISSEMENT UTILISANT DES PROTEINES MORPHOGENIQUES OSSEUSES

(30) Priority: 21.02.2001 US 270528 P; 09.11.2001 US 348111 P
(43) Date of publication of application: 25.02.2004
(73) Proprietor: STRYKER CORPORATION, Kalamazoo, Michigan 49005 (US); Rush University Medical Center, Chicago, IL 60612 (US)
(72) Inventor: CHUBINSKAYA, Susanna, Vernon Hills, IL 60061 (US); RUEGER, David, C., Southborough, MA 01772 (US); KUETTNER, Klaus, E., Chicago, IL 60657 (US)
(74) Representative: Kirkham, Nicholas Andrew
(86) International application number: PCT/US2002/005551
(87) International publication number: WO 2002/068962

(56) References cited:
- WO-A-96/39431
- WO-A-98/54572
- US-A- 4 857 456
- US-A- 5 707 810
- US-B1- 6 194 376
- BAUER D C ET AL: "BIOCHEMICAL MARKERS OF BONE TURNOVER AND PREDICTION OF HIP BONE LOSS IN OLDER WOMEN: THE STUDY OF OSTEOPOROTIC FRACTURES" JOURNAL OF BONE AND MINERAL RESEARCH, NEW YORK, NY, US, vol. 14, no. 8, 1999, pages 1404-1410, XP002952591 ISSN: 0884-0431

## Description

### Field of the Invention

The invention relates generally to uses of OP-1 and other bone morphogenic proteins as biomarkers of tissue integrity or deterioration, and more particularly to methods for diagnosing and/or monitoring cartilage degeneration associated with inflammatory disease and age.

### Related Applications

### Background of the Invention

A number of factors can cause or contribute to cartilage degeneration in mammals, including trauma and inflammatory disease. Damage to cells resulting from the effects of inflammatory response has been implicated as the cause of reduced cartilage function or loss of cartilage function in diseases of the joints (e.g., rheumatoid arthritis (RA) and osteoarthritis (OA)). In addition, autoimmune diseases such as systemic lupus erythematosis (SLE) and scleroderma can also be characterized by a degradation of connective tissue. In the case of some cartilage degenerative diseases such as osteoarthritis (OA), the mechanisms that turn the normal aging of articular cartilage into the pathological OA process are currently unknown.

OA is a debilitating joint disease affecting primarily the elderly. OA is rare in young adults, starts to be developed symptomatically and radiographically in adults by their late 40's and 50's, and then rapidly increases in prevalence from age 60 to 70 years (Hamerman (1993) J. Am. Geriatr. Soc. 41:760). However, in general, as any biological tissue or organ ages, function gradually declines and susceptibility to disease and injury increases (Buckwalter et al. (1993) JBJS 75-A:1533). Age-related changes in tissue biosynthesis include 1) an increased denaturation of collagen type II (Hollander et al. (1995) J. Clin. Invest. 96(6):2859); 2) a decline in the synthesis of DNA (Ribault et al. (1998) Mechan. Ageing & Devel. 100(1):25), proteoglycans, and link protein (DeGroot et al. (1999) Arthrit. Rheum. 42(5):1003; Iqbal et al. (2000) Biochem. Biophys. Res. Commun. 274(2):467; Verbruggen et al. (2000) Osteoarthr. Cartil. 8(3):170; Bolton et al. (1999) Biochem. J. 337(Pt 1):77); 3) an increased sulfation of chondroitin sulfate (Brown et al. (1998) Am. J. Vet. Res. 59(6):786); 4) an accumulation of hyaluronan (Platt et al. (1998) Equine Vet. J. 30(1):43) and cartilage intermediate layer protein (Lorenzo et al. (1998) J. Biol. Chem. 273(36):23463); 5) structural changes in fibromodulin (Roughley et al. (1996) Osteoarthr. Cartil. 4(3):153); 6) a decreased ability to assemble large molecular size aggregates (Verbruggen et al. 2000); 7) elevated levels of transglutaminase activity, which promotes pathologic matrix mineralization and cartilage degeneration (Rosenthal et al. (1997) Arthrit. Rheum. 40:966); and 8) increased apoptosis (Adams and Horton (1998) Anatom. Rec. 250(4):418).

Importantly, with aging the responsiveness of articular cartilage to different growth factors, such as transforming growth factor-β (TGF- β) (Iqbal et al. 2000; Guerne et al. (1995) Arthrit. Rheum. 38(7):960), insulin-like growth factor-1 (IGF-1) (Messai et al. (2000) Mechan. Ageing & Devel. 115(12):21), epidermal growth factor (EGF) (Ribault et al. 1998), osteogenic protein-1 (OP-1) (Flechtenmacher et al. (1996) Arthrit. Rheum. 39:1896) and others, is also altered.

While the underlying causes of articular cartilage degeneration seen with age or due to inflammatory disease have not been identified, there is increasing evidence that growth factors (especially those expressed endogenously in cartilage) and cytokines play a critical mediatory role. For example, the bone morphogenetic protein (BMP) family of growth factors are important regulators of matrix production that can also inhibit certain degradative processes. BMPs were originally identified as proteins capable of inducing ectopic endochondral bone formation in subcutaneous implants (Urist et al. (1979) Proc. Natl. Acad. Sci. USA 76:1828; Sampath and Reddi (1981) Proc. Natl. Acad. Sci. USA 78:7599). Subsequent molecular cloning revealed that the BMP family consists of a large number of related molecules that belong to the TGF- β superfamily. Although BMPs were initially found in the bone matrix, it is now clear that they are expressed in a variety of tissues.

Osteogenic Protein 1 (OP-1) is the seventh member of the BMP family (BMP-7). It is synthesized as a large precursor, approximately three times larger than a mature protein, and is ultimately processed proteolytically at the C-terminal region to yield a mature disulfide-linked dimer. OP-1 is most closely related to BMP-6 and BMP-5 (88% and 87% homology, respectively), and to a lesser extent to BMP-2 and BMP-4 (60% and 58% homology, respectively), with some homology to BMP-3 (42% homology) and TGF-β (about 30% homolog) (Cook and Rueger (1996) Clin. Orthoped. Rel, Res. 324:29; Sampath and Rueger (1994) Complicat. Orthoped. Winter:101). Originally, OP-1 was purified from bovine demineralized bone (Sampath et al. (1990) J. Biol. Chem. 265(22):13198) with its recombinant form being subsequently cloned from human cDNA libraries in Chinese hamster ovary (CHO) cells (Özkaynak et al. (1990) EMBO J. 9:2085).

US-A-4857456 discloses assays of bone Morphogenetic protein (BMP) and anti-BMP antibody in the diagnosis of bone disorders.

US-A-5707810 discloses methods of diagnosing renal tissue damage or disease.

J. Bone and Mineral Research, vol.14, No.8, 1999 pages 1404-1410 (Bauer et al) discloses biochemical markers of bone turnover and prediction of hip bone loss in older women: the study of osteoporotic fractures.

WO-A-9639431 discloses bone morphogenic protein-10.

WO-A-9854572 discloses methods for evaluating tissue morphogenesis and activity.

The critical importance of BMPs for cartilage and bone formation was demonstrated using the transgenic approach: lack of some BMP genes caused skeletal abnormalities and eventually the lethality of mouse embryos (Dudley et al. (1995) Genes Dev. 9:2795; Luo et al. (1995) Genes Dev. 9:2808). Recent studies have focused on the potential role of exogenous OP-1 in human and bovine cartilage homeostasis and repair. (Flechtenmacher et al. 1996; Huch et al. (1997) Arthrit, Rheum. 40:2157; Koepp et al. (1999) Inflamm. Res. 47:1; Nishida et al. (2000) Arthrit. Rheum. 43:206). These studies showed that human recombinant OP-1 (rhOP-1) caused a significant anabolic response in articular cartilage. It induced the synthesis of major matrix components aggrecan and collagen type II in human chondrocytes of different ages with continued expression of the chondrocyte phenotype (Flechenmacher et al. 1996; Huch et al. 1997). In addition, OP-1 has been shown to induce the synthesis of hyaluronan, its receptor CD44 and hyaluronan synthase-2, to promote the formation and retention of the extracellular matrix (Nishida et al. 2000) and to counteract catabolic events, such as interleukin-1 (IL-1), fibronectin (FN-f) and collagen fragment-induced cartilage degeneration (Huch et aL 1997; Koepp et al, 1999; Jennings et al. (2001) Connect. Tiss. Res. 42(1):71-86. When the effect of OP-1 on FN-f-challenged cartilage was compared to that of TGF-β, it was found that TGF-5 was not only able to block FN-f mediated proteoglycan (PG) depletion, but by itself promoted a decrease in cartilage PG content (Koepp et al. 1999). Importantly, rhOP-1 did not lead to chondrocyte proliferation and differentiation in human and bovine adult articular cartilage (Flechtenmacher et al. 1996, Chen et al. (1993) Biochem. Biophys. Res, Commun. 197:1253).

It has recently been demonstrated that OP-1 is endogenously expressed in human adult articular chondrocytes (Chubinskaya et al. (2000) J. Histochem. Cytochem. 48(2):239). Moreover, in human articular cartilage, OP-1 is present in two forms: the unprocessed, pro-form, and the processed, mature-form. Mature OP-1 was immunolocalized primarily in the superficial layer of cartilage, while pro-OP-1 was detected in the deep layer. The endogenous expression of OP-1 by articular chondrocytes indicates that articular cartilage has the potential to repair and might suggest the unique role of this BMP in tissue protection and regeneration. This is supported by recent data demonstrating that over-expression of OP-1 in mice led to the increased synthesis of matrix macromolecules, collagen type II and PGs (Hidaka et al. (2000) Trans. ORS 46:41).

Diagnostic assays for RA include Rose's method based on the detection of rheumatoid factor, modified Rose's method by Heller, the RAHA-test, and the RA-test. These methods, however, possess disadvantages in that blood rheumatoid factor is not specific to patients with RA. Rheumatoid factor assay kits based on such methods also have poor accuracy and reproducibility.

Assays for erythrocyte sedimentation rate (ESR) or C-reactive protein (CRP) are useful for determining the activity of inflammatory diseases such as RA but are not suitable for use in their diagnosis. Detection of anti-nuclear antibodies or LE cells may be used to detect RA but it is difficult to accurately diagnose RA because these methods are not specific for RA and because such antibodies or cells are frequently detectable in other collagen diseases. In addition, these methods do not specifically detect the cartilage degradation which is associated with RA.

A need therefore exists for a biochemical marker which can be used to specifically and reproducibly detect the presence of, or predisposition to acquiring, cartilage degeneration and destruction.

### Summary of the Invention

The invention relates generally to methods for determining tissue integrity using bone morphogenic proteins such as OP-1 as a biomarker. Specifically, the invention relates to methods for determining the health or ill-health of tissues such as cartilage (e.g., degradation, deterioration or regeneration) in a patient by measuring the level of OP-1 protein and/or OP-1 mRNA in a patient tissue sample. In particular, the invention is based on the discovery that OP-1 is a biomarker for inflammation associated, autoimmune, and age-related tissue changes such as cartilage degradation.

In one aspect, the invention relates to methods for detecting, diagnosing, determining a predisposition for, or monitoring cartilage degradation in a patient due to inflammation. In one embodiment, the invention provides a method of determining the presence of an inflammatory disease in a patient by determining the amount of OP-1 protein and/or OP-1 mRNA present in a joint tissue sample of the patient and comparing this amount to a predetermined standard. The predetermined standard may comprise a range of OP-1 protein and/or OP-1 mRNA concentration values and may be an age-adjusted standard. The difference in OP-1 protein or OP-1 mRNA levels in the sample and the predetermined OP-1 protein or OP-1 mRNA standard may be indicative of the presence (or absence) of an inflammatory disease. The joint tissue sample tested may be, for example, cartilage, ligament, meniscus, tendon, synovium, synovial fluid or intervertebral disc tissue. OP-1 protein is preferably measured using an enzyme-linked immunosorbent assay (ELISA). OP-1 mRNA is preferably measured using a reverse transcription polymerase chain reaction (RT-PCR). In a preferred embodiment, the methods according to the invention are used to determine the presence of an inflammatory disease such as, for example, an autoimmune disease (e.g., rheumatoid arthritis, lupus erythematosus and non-inflammatory monoarthritis), gout, fibromyalgia syndrome, and polymyalgia rheumatica. In one embodiment, disease is associated with a histomorphological change in joint tissue. The histomorphological change in a joint tissue may be indicative of a degenerative, autoimmune, inflammatory connective tissue, or trauma-induced disease. The histomorphological change in a joint tissue may also be indicative of regenerative and reparative processes in the joint.

In another embodiment, the invention provides a method of determining the clinical severity of an inflammatory disease in a patient, by determining the amount of OP-1 protein and/ or OP-1 mRNA present in a joint tissue sample and comparing this amount to a predetermined statistical relationship. The predetermined statistical relationship is based on a comparison of levels of OP-1 protein and/or OP-1 mRNA obtained from members of a population having different clinical severities of an inflammatory disease. The severity of the disease as measured by OP-1 protein and/or mRNA may or may not correlate with the clinical findings, i.e., for example, a patient may appear or feel normal but may manifest altered OP-1 protein and/or mRNA levels, thereby indicating the existence or predisposition to an inflammatory disease. Similarly,m the existence or predisposition to an age-related disease can be determined.

In another embodiment, the invention provides a method of determining the predisposition for an inflammatory (e.g., autoimmune) disease in a patient, by determining the amount of OP-1 protein and/or OP-1 mRNA present in a joint tissue sample of a patient and comparing this amount to a predetermined standard. The predetermined standard may comprise a range of OP-1 protein and/or OP-1 mRNA concentration values and may be an age-adjusted standard. The difference in OP-1 protein and/or OP-1 mRNA levels in the sample and the predetermined standard is indicative of a predisposition for developing an inflammatory (e.g., autoimmune) disease.

In another aspect, the invention relates to methods for detecting, diagnosing, determining a predisposition for, or monitoring cartilage degradation in a patient due to an age-related disorder or a disorder characterized by accelerated or abnormal tissue aging. In an embodiment, the invention provides a method of determining the presence of an age-related tissue disorder or a disorder characterized by accelerated or abnormal tissue aging in a patient by determining the amount of OP-1 protein and/or OP-1 mRNA present in a joint tissue sample and comparing this amount to a predetermined standard. The difference in OP-1 protein and/or OP-1 mRNA levels in the sample and the predetermined standard is indicative of the presence of an age-related disorder or a disorder characterized by abnormal tissue aging. The joint tissue sample tested may be cartilage, ligament, meniscus, tendon, synovium, synovial fluid or intervertebral disc tissue. OP-1 protein is preferably measured using an enzyme-linked immunosorbent assay (ELISA). OP-1 mRNA is preferably measured using reverse transcription polymerase chain reaction (RT-PCR). In a preferred embodiment, the methods according to the invention are used to detect, diagnose, predict or monitor cartilage degradation due to an age-related disorder or a disorder characterized by abnormal tissue aging such as, for example, osteoporosis or osteoarthritis. In an embodiment, the age-related tissue disorder is independent of chronological age. In another embodiment, the predetermined standard is age-correlated.

In another embodiment, the invention provides a method of determining the clinical severity of an age-related tissue disorder or a disorder characterized by accelerated or abnormal tissue aging by determining the amount of OP-1 protein and/ or OP-1 mRNA present in a joint tissue sample and comparing this amount to a predetermined statistical relationship. The predetermined statistical relationship is based on a comparison of levels of OP-1 protein and/or OP-1 mRNA obtained from members of a population having different clinical severities of an age-related tissue disorder or a disorder characterized by abnormal tissue aging.

In another embodiment, the invention provides methods of determining the predisposition for an age-related tissue disorder, a disorder characterized by accelerated or abnormal tissue aging, an inflammatory disease, an autoimmune disease, a joint degenerative disease, or a joint trauma-induced disease in a patient by determining the amount of OP-1 protein and/or OP-1 mRNA present in a joint tissue sample and comparing this amount to a predetermined standard. The predetermined standard may comprise a range of values and/or be an age-adjusted standard. The difference in OP-1 protein and/or OP-1 mRNA levels in the sample and the predetermined standard is indicative of a predisposition for an age-related tissue disorder or disorder characterized by abnormal tissue aging.

In another aspect, the invention provides methods of determining the clinical or disease status of a joint region in a patient, by determining the amount of OP-1 protein and/or OP-1 mRNA present in a patient tissue sample from a joint region and comparing this amount to a predetermined standard. The predetermined standard may comprise a range of OP-1 protein and/or OP-1 mRNA concentration values and/or be an age-adjusted standard. The OP-1 protein and/or OP-1 mRNA levels in the sample are compared to the amount of OP-1 protein and/or OP-1 mRNA in the predetermined standard to determine a value representative of the deviation of the patient's levels with the standard, the value being indicative of the clinical status of the patient's joint region. In an embodiment, the predetermined standard is correlated with the age of the patient and is representative of an amount of OP-1 protein and/or OP-1 mRNA expected to be present in a clinically-normal joint region. In another embodiment, the predetermined standard has a range of values.

In another embodiment, the invention provides methods for monitoring degenerative or regenerative activity within a joint region of a patient by determining OP-1 protein and/or OP-1 mRNA levels in a tissue sample obtained from a joint region of a patient at a certain time point, determining the amount of OP-1 protein and/or OP-1 mRNA present in a tissue sample obtained from the joint region of a patient at a second, later time point, and comparing OP-1 protein and/or OP-1 mRNA levels at the second time point to those of the first time point. An increase in the amount of OP-1 protein and/or OP-1 mRNA present is indicative of an onset of or increase in regenerative activity in the joint region, and a decrease in the amount of OP-1 protein and/or OP-1 mRNA present in the joint region is indicative of a cessation of, or decrease in, regenerative activity in the joint region.

In another embodiment, the invention provides a method of determining the clinical status of a joint region of a patient by determining the amount of OP-1 protein and/or OP-1 mRNA present in a tissue sample obtained from a joint region of a patient and comparing it with a predetermined standard indicative of OP-1 protein and/or OP-1 mRNA levels expected to be present in a clinically normal joint region. The amount of OP-1 protein and/or mRNA present in the tissue sample that is about equal to the standard is indicative of a normal clinical status of the joint region, and an amount that is not about equal to the standard is indicative of an abnormal clinical status of the joint region of said patient.

In another aspect, the invention provides methods for determining the effective dose of an anti-inflammatory agent in a subject by administering to a subject a dose of an anti-inflammatory agent, obtaining a tissue, body fluid or cell sample from the subject, determining OP-1 protein concentration or OP-1 mRNA concentration in the sample, determining the concentration of protein or mRNA encoded by a second gene whose expression is not altered by inflammation; and comparing the OP-1 protein or mRNA concentration to the protein or mRNA concentration of the second gene, wherein the difference between the OP-1 protein or mRNA concentration and the second gene protein or mRNA concentration is indicative of an effective increase or decrease in OP-1 protein or mRNA concentration and thus the effectiveness of the anti-inflammatory agent dose in the patient.

In another embodiment, the invention provides methods for determining the ability of a patient to respond to an anti-inflammatory agent by administering to a subject a dose of an anti-inflammatory agent, obtaining a tissue, body fluid or cell sample from the subject to whom a dose of an anti-inflammatory agent was earlier administered, determining the OP-1 protein concentration or OP-1 mRNA concentration in the sample, determining in the same sample the concentration of protein or mRNA encoded by a second gene whose expression is not altered by inflammation, and comparing the OP-1 protein or mRNA concentration to the protein or mRNA concentration of the second gene to create a ratio, wherein the subject is responsive to anti-inflammatory agents if the ratio is higher than a predetermined control ratio for untreated or nonresponsive subjects, or similar to prior ratios for the subject when the subject was previously determined to be responsive.

### Brief Description of the Drawings

The foregoing and other objects, features and advantages of the present invention, as well as invention itself, will be more fully understood from the following description of preferred embodim when read together with the accompanying drawings, in which:
Figure 1 is an exemplary standard curve generated by the OP-1 sandwich ELISA, prepared using human recombinant mature OP-1 at different dilutions ranging from 0.01 to 10 ng/ml.
Figure 2 illustrates the effect of a wide range of pHs for TBS/Tween buffer on the measurement of various concentrations of OP-1 in the OP-1 sandwich ELISA.
Figure 3 illustrates the effect of 1M GuHCl extraction buffer (A) and lysis extraction buffer (B) on the standard curve generated by the OP-1 sandwich ELISA. Closed squares represent OP-1 diluted in TBS buffer; opened circles represent OP-1 diluted either in GuHCl buffer (A) or in lysis (B) buffer.
Figure 4A illustrates the level of OP-1 protein in cartilage samples from normal individuals of different ages analyzed with OP-1 sandwich ELISA.
Figure 4B illustrates the level of OP-1 protein in cartilage samples from normal individuals whose cartilage also had normal histomorphology, analyzed with OP-1 sandwich ELISA.
Figure 5 represents a semi-quantitative image analysis of Western Blot bands that correspond to the mature OP-1 dimer (36 kD) and the OP-1 hemidimer (75 kD).
Figure 6 illustrates a quantitative image analysis of Western Blot bands that correspond to the hemidimer form of pro-OP-1.
Figure 7 illustrates semi-quantitative PCR of OP-1 mRNA/GADPH mRNA ratios from cartilage samples plotted against the age of the donors.
Figure 8 illustrates an exemplary standard curve for the OP-1 Sandwich ELISA.
Figure 9A illustrates OP-1 protein concentration in patients with cartilage Collins grades of 0-4 and osteoarthritis (OA) patients.
Figure 9B illustrates OP-1 mRNA concentration in patients with cartilage Collins grades of 0-4 and osteoarthritis (OA) patients.
Figure 10 illustrates the level of OP-1 protein in human synovial fluid from normal, OA, and rheumatoid arthritis (RA), as well as other patients with various arthritic diseases, such as gout, fibromyalgea syndrome (FMS), and polymyalgea rheumatica (PMR).
Figure 11 illustrates the amount of total protein in synovial fluid from normal, OA, RA, as well as other patients with various arthritic diseases, such as gout, fibromyalgea syndrome (FMS), and polymyalgea rheumatica (PMR).
Figure 12 illustrates the level of OP-1 protein normalized to total protein concentration in synovial fluid from normal, OA, and RA, as well as other patients with various arthritic diseases, such as gout, fibromyalgea syndrome (FMS), and polymyalgea rheumatica (PMR).
Figure 13A illustrates the level of OP-1 protein in normal cartilage, ligament, tendon, meniscus, and synovium.
Figure 13B illustrates the level of OP-1 mRNA in normal cartilage, ligament, tendon, meniscus, and synovium.
Figure 14 illustrates the level of OP-1 protein expression in cartilage cultures from normal donors in response to low dose of IL-1β. After a 4 day equilibration period (culture in the presence of media only), explants were treated with 0.1ng/ml of IL-1β for 48 or 96 hours.
Figure 15 illustrates the recovery of endogenous OP-1 protein after removal of a low dose of IL-1β. Cartilage samples were cultured first for 48 hours in the presence of 0.1ng/ml IL-1β, the IL-1β was removed and cartilage was cultured for an additional 48 hours in media only.
Figure 16 illustrates the level of OP-1 protein expression in cartilage cultures from normal donors in response to high dose of IL-1β. After a 4 day equilibration period (culture in the presence of media only), explants were treated with 1.0ng/ml of IL-1β for 48 or 96 hours.
Figure 17 illustrates changes in endogenous OP-1 protein levels in cartilage cultures after removal of high dose IL-1β. Cartilage samples were cultured first for 48 hours in the presence of 1.0ng/ml IL-1β, the IL-1β was removed and cartilage was cultured for an additional 48 hours in media only.

### Detailed Description of the Invention

The invention is based on the discovery that OP-1 protein and mRNA levels decrease as a consequence of normal aging and in response to inflammation. OP-1 protein and mRNA levels in cartilage decrease with increasing age of a patient regardless of the presence of observable cartilage degradation. In addition, OP-1 protein and mRNA levels are decreased in inflamed tissue (e.g., inflamed joint tissue such as cartilage). Similar results are obtained when synovial fluid is tested.

The invention is based on the discovery that the concentration of an exemplary bone morphogenic protein, OP-1, or its mRNA in cartilage and synovial fluid is decreased in normal aging and around inflamed tissue (e.g., joint tissue). Two methods were applied for the quantification of the levels of endogenous OP-1 protein in these samples, a sandwich ELISA and Western Blot, however, any method for the quantification of protein may be used. Cartilage tissue was lyophilized and OP-1 protein was extracted. Human recombinant mature OP-1 and monoclonal and polyclonal OP-1 antibodies were used in the ELISA and the results were normalized to the dry weight of the tissue sample. The same antibodies were used in Western Blot analysis. The densities of specific immunoreactive bands were analyzed with a Fluor-S Multilmager. Western Blot results were normalized to the total protein content.

OP-1 mRNA expression was measured by using routine nested RT-PCR on total RNA from connective tissues or synovial fluid; however any method for quantifying OP-1 mRNA may be used and any of a number of tissues may be tested, such as those connective tissues which are exposed to an affected locus (e.g., an orthopedic site such as a knee, elbow or knuckle joint which is inflamed and/or in which cartilage is degraded). Densities of the RT-PCR bands were evaluated using a Fluor-S Multilmager with attached software program and were normalized to the densities of control GAPDH mRNA bands; however, the values of OP-1 mRNA may be normalized to the RT-PCR product of any mRNA whose concentration is not altered (i.e., not upregulated or downregulated) by inflammation or age. Any of a number of PCR products may be generated using different PCR primers capable of amplifying OP-1 mRNA or a second gene mRNA used as a normalization control.

In a preferred embodiment, RNA levels are quantified by amplification of the RNA by reverse transcription polymerase chain reaction (RT-PCR) of the RNAs. The reaction products may be resolved/quantified, e.g., by gel electrophoresis (e.g., slab or capillary) or the unamplified RNA may be quantified, e.g., by scanning laser, Northern blot analysis, or by direct hybridization with a probe. Alternatively, RNA levels are quantified by *in situ* detection. In an embodiment of the invention, probes capable of hybridizing specifically to OP-1 mRNA are attached to a solid phase support, e.g., a "chip" or "DNA probe array". Oligonucleotides can be bound to a solid support by a variety of processes, including lithography. For example, a chip can hold up to about 250,000 oligonucleotides. The solid phase support is then contacted with a test nucleic acid and hybridization to the specific probes is detected. Accordingly, the quantification of numerous samples (e.g., different tissues from the same individual or samples from different individuals) can be carried out in a single hybridization experiment.

Diagnostic protein or mRNA procedures may also be performed *in situ* directly upon sections (fixed or frozen) of tissue obtained from biopsies or resections by looking at relative intensities of OP-1 protein and/or mRNA and control protein and/or RNAs (e.g., GADPH) in a portion of the biopsy sample, such that no protein or nucleic acid purification is necessary. Nucleic acid reagents or antibodies may be used as probes and/or primers for such *in situ* procedures.

RNA may be quantified from any tissue, including an organ, body fluid or nucleated cell. For example, the tissue is preferably cartilage and the body fluid is preferably synovial fluid or blood. The tissue is obtained and is preferably stored in a stabilization solution or is stored frozen prior to analysis to minimize RNA and protein degradation. In an embodiment, the tissue is derived from a joint (i.e., a joint tissue or joint region), such as the tissue of the knee, elbow, shoulder, hip, thigh, neck, vertebrae, knuckle, finger, wrist or ankle, for example.

As contemplated herein, tissue can itself be inflamed or body fluid is considered inflamed when situated adjacent to or when physiologically related to inflamed tissue, or otherwise contacted with inflamed tissue or an inflamed anatomical structure. Inflammation is typified by, to name but a few, redness, swelling, fever, and/or pain and/or the extravasation of plasma and infiltration of leukocytes into the site of an insult or trauma. The inflammation or inflammatory disease can be caused by any insult or trauma which causes the body to respond by mounting a protective response (e.g., immune response) which causes redness, swelling, fever, and/or pain and/or the extravasation of plasma and infiltration of leukocytes into the site of the insult or trauma. The insult or trauma can be due to injury, infection, allergy, disease or surgery, for example. In certain preferred embodiments, the insult which causes inflammation or an inflammatory disease can be genetic in nature, thus predisposing an individual or making an individual particularly susceptible to an inflammatory disease. The inflammation or inflammatory disease can be hyperacute (peracute), acute, subacute, or chronic inflammation. The degree of tissue damage can be superficial or profound, nonspecific, or specific to a particular tissue. The immunopathogenic mechanism behind the inflammation can be allergic (reaginic), mediated by cytotoxic antibodies, mediated by immune complexes, or a delayed-type hypersensitivity reaction, for example.

The levels of OP-1 protein and/or OP-1 mRNA may be compared to a predetermined standard control level which corresponds to a particular disease, a particular stage of a disease, a particular severity of a disease, or a particular tissue grade (e.g., Collins grade). Comparison with the levels of OP-1 protein and/or OP-1 mRNA in the patient sample with the predetermined standard values for OP-1 protein and OP-1 mRNA at a particular stage or severity of a disease or tissue grade is an indication of the stage, severity or tissue grade, respectively. Alternatively, the OP-1 protein and/or OP-1 mRNA levels in a patient may be compared with levels in that patient which were determined prior to the onset of disease or during remission of the disease. The standard may be an age-adjusted standard, i.e., a standard which is derived from the measurements of tissue samples from a particular age-group and which values are representative of that age group.

The severity of disease refers to the intensity of symptoms or manifestations of the disease, such as pain, swelling, edema, redness, tissue degradation, alterations in the biosynthetic activity of the involved tissue (e.g., increase or decrease in the synthesis of inflammatory mediators or other proteins). The severity of disease can also correlate to predetermined levels of severity of illness within a diagnostic group which are established by various measurement criteria (i.e., a severity of illness index such as the widely-used Collins grading system). Clinical disease refers to a disease which presents with specific clinical signs and symptoms that are recognizable, as distinct from a subclinical illness without clinical manifestations. A predisposition to a disease refers to latent susceptibility to disease which can be activated under certain conditions, as by stress, age or injury. A predisposition can refer to the likelihood of acquiring a disease state at some point in time, regardless of the onset of clinical symptoms of the disease. A predisposition can be a genetic predisposition, which disease is not present in youth but can manifest itself later in life.

The invention provides methods for monitoring a subject's response to an anti-inflammatory agent by administering to a subject a dose of an anti-inflammatory agent, obtaining a tissue, body fluid or cell sample from the subject, determining the level of expression of the OP-1 gene (either protein and/or mRNA) and comparing OP-1 gene expression pre- and post-treatment to determine whether the subject is responsive to the anti-inflammatory agent, e.g., has normal OP-1 gene expression. Alternatively, the invention provides methods for monitoring a subject's response to an OP-1 modulating agent (e.g., an agent that modulates the RNA and/or protein expression of OP-1) by administering to a subject a dose of an OP-1 modulating agent, obtaining a tissue, body fluid or cell sample from the subject, determining the level of expression of the OP-1 gene (OP-1 protein and/or mRNA) and comparing OP-1 gene expression pre- and post-treatment to determine whether the subject is responsive to the OP-1 modulating agent.

In another embodiment, the invention provides methods for determining drug responsiveness in a tissue, including a body fluid or cell, after exposure *in vitro* to an anti-inflammatory agent or OP-1 modulating drug. In yet another embodiment, the invention provides methods for determining OP-1 protein or mRNA levels or drug responsiveness in a tissue, body fluid or cell of an animal such as a mammal (e.g., a mouse, rat, rabbit, pig, goat, dog, cow, horse, cat). In an embodiment, the animal is a transgenic animal or disease model animal (e.g., a goat model for rheumatoid arthritis). The animal may be analyzed for OP-1 protein and/or mRNA levels after administering an anti-inflammatory agent or OP-1 modulating drug to the animal.

It is contemplated that other members of the BMP family of proteins can be used as a biomarker for the disease or age status of skeletal tissue and joint tissue as disclosed herein for an exemplary member, OP-1. The RT-PCR methods can be altered by designing primers for amplifying other BMP mRNAs. Detailed descriptions of other members of the BMP family of proteins related structurally and biochemically to OP-1, as well as corresponding amino acid and nucleotide sequences therefor, can be found in the art, for example, in US 5,011,691 issued on 4-30-91, US 5,258,494 issued on 11-2-93, US 5,324,819 issued on 6-28-94, US 5,750,651 issued on 5-12-98, US 5,266,683 issued on 11-30-93, US 5,863,758 issued on 1-26-99, US 6,262,835 issued on 7-17-01. In addition, the ELISA and Western Blot methods described herein may be readily adapted using routine experimentation and used to measure other BMP proteins. Similarly, exemplary teachings relating to antibodies for detecting BMPs as well as for preparing such antibodies can be found in US 5,468,845 issued on 11-21-95 and US 5,714,589 issued on 2-3-98.

Methods according to the invention are particularly useful for predicting, determining, measuring or monitoring a subject who suffers from, or is predisposed to suffering from, a disease such as, for example, osteoarthritis, rheumatoid arthritis, psoriatic arthritis or idiopathic arthritis. Methods according to the invention are also useful for predicting, determining, measuring or monitoring a subject who suffers from, or is predisposed to suffering from, an autoimmune disease, such as systemic lupus erythematosus. Other diseases and conditions that have an inflammatory component or consequence include, but are not limited to, autoimmune arthritis, juvenile rheumatoid arthritis, psoriatic arthritis, gingival inflammation, inflammation due to periodontal disease, gout. Other diseases which have an inflammatory component include bacterial infections, viral infections, and fungal infections. It is likely that the differences between OP-1 protein and mRNA levels in diseased and normal tissue might vary in magnitude, or OP-1 protein and/or mRNA levels might be higher or lower than normal, depending on the disease type, the severity of the disease or the stage of the disease.

Notwithstanding the foregoing, it is understood that the present invention distinguishes between tissue deterioration such as cartilage degeneration or degradation which can accompany inflammation and tissue deterioration such as cartilage degeneration or degradation which can occur independent of inflammation or disease.

That is, OP-1 protein or mRNA levels can be an indicia of tissue integrity or health but not necessarily an indicia of an underlying cause of tissue deterioration or ill-health. For example, as disclosed herein, OP-1 is an indicia of cartilage degeneration which accompanies inflammatory joint disease as well as an indicia of age-related cartilage deterioration which is independent of disease.

### Exemplification

### Example 1: OP-1 protein and mRNA Levels Decrease with Increased Age

The changes in endogenous OP-1 (protein and mRNA) expression with aging of human articular cartilage were studied. In order to assess quantitatively the concentration of total endogenous OP-1 protein in cartilage extracts, a sandwich enzyme-linked immunosorbent assay (ELISA) was developed and compared with Western Blot and reverse transcription polymerase chain reaction (RT-PCR) measurements. Results indicate that there is a correlation between a decrease in total and mature OP-1 protein and OP-1 mRNA with increased age.

### Materials and Methods

### Reagents

Human recombinant pro- and mature-OP-1, BMP-6, anti-pro (R2854) and anti-mature (1B12) OP-1 antibodies were obtained from Stryker Biotech (Hopkinton, MA). Two other, anti-OP-1 antibodies (#SC-9305 and #MAB354) were purchased from Santa Cruz Biotechnology, Inc. (Santa Cruz, CA) and R&D Systems (Minneapolis, MN), respectively. Electrophoresis grade reagents were purchased from Bio-Rad (Hercules, CA). Chemicals, either reagent or molecular biology grade, were obtained from Sigma Chemical Co. (St. Louis, MO) unless otherwise noted. Keratanase (Pseudomonas sp.; EC 3.2.1.103), keratanase II (Bacillus sp. Ks 36) and chondroitinase ABC (Proteus vulgaris; EC 4.2.2.2) were obtained from Seikagaku, Japan. Hyaluronidase (bovine testicular) was purchased from Sigma (St. Louis, MO).

### Tissue acquisition

Full thickness normal human articular cartilage was dissected from load bearing regions of femoral condyles of donors with no history of joint disease within 24 hours of death. Samples from men and women ranging from 20 to 80 years old were obtained with institutional approval through the Regional Organ Bank of Illinois according to their protocol. After opening the joint, the surface of the cartilage was subjected to gross examination. Although all cartilage samples were obtained from normal donors, not all of them appeared to be normal. Some samples revealed degenerative morphological changes. All cartilage samples were processed for either messenger RNA or protein extraction.

### OP-1 antibodies

The following antibodies were used: a polyclonal antibody, R2854 (Stryker Biotech), specific for the pro- form of OP-1 (Chubinskaya et al. 2000; Jones et al. (1994) Growth Fact. 11:215; Helder et al. (1998) J. Dent. Res. 77:545; Vukicevic et al. (1994) Biochem. Biophys. Res. Commun. 198:693); two monoclonal antibodies, 1B12 (Stryker Biotech) and #MAB354 (R&D Systems), specific for the entire mature domain of OP-1; and a polyclonal antibody, #SC-9305 (Santa Cruz) specific for a 15 amino acid synthetic peptide derived from the N-terminus of mature OP-1. Initially, the specificity of all antibodies was tested by the suppliers. However, considering the high degree of homology between OP-1 protein and BMP-6 protein and the fact that BMP-6 was cloned after the anti-OP-1 antibodies were produced, all antibodies were tested and non-cross-reactivity with BMP-6 was confirmed. Anti-pro-OP-1 antibody, R2854, showed no specific binding to either BMP-6 or mature OP-1.

### Cartilage extraction

500 mg of fresh donor cartilage was lyophilized overnight and the dry weight of the tissue was measured. Samples were pulverized in liquid nitrogen and 150 mg (dry weight) of cartilage tissue was extracted with 3.5 ml of ice-cold 1M GuHCl buffer, pH 7.5, containing 10 mM CaCl₂, 50 mM Tris, and 1 tablet/10mls of protease inhibitor (Roche Diagnostics # 1836153, Indianapolis, IN). Cartilage extraction was performed at 4°C for 4 hours with rotation. Supernatants were centrifuged at 2500 rpm for 10 min at 4°C and stored at 4°C. Supernatants were dialyzed for 2 days in water (12,000-14,000 MW cut off) and stored at 4°C. In order to prove the efficiency of the extraction, the cartilage tissue was extracted again after the supernatants were removed and the extracts were analyzed by Western blot and ELISA.

Collins grade 0 generally relates to cartilage in which there is no cartilage degeneration or osteophytes. Collins grade 1 generally relates to cartilage in which there is limited disruption of the articular surface and minor fibrillations. Collins grade 2 generally relates to cartilage in which there is fibrillation of cartilage with fissures, and perhaps some small osteophytes. Collins grade 3 generally relates to cartilage in which there is extensive fibrillation and fissuring, about 30% or less of the cartilage surface is eroded down to subchondral bone (focal lesions) and osteophytes are present. Collins grade 4 generally relates to cartilage in which greater than 30% of the cartilage surface is eroded down to the subchondral bone, with gross geometric changes, and osteophytes are present.

### Western Blot Analysis

Immunoblot analysis was performed with the anti-pro (R2854) and anti-mature (1B12) OP-1 antibodies described above. The lyophilized samples were solubilized in a buffer containing 10 mM Tris, pH 6.5, 1% SDS, 10% Glycerol, and 0.016% Bromphenol Blue. The samples were reduced with 10 mM dithiothreitol (DTT). Protein concentration was quantified by Micro BSA Protein Assay Reagent Kit (Pierce, Rockford, IL). Thirty µg of each cartilage sample was loaded onto 12% SDS-PAGE gels, electrophoresed, and Western blotted according to standard methods. Non-specific binding sites were blocked with blocking solution containing 5% milk (Bio-Rad, Hercules, CA) for 1 hour. The blots were incubated with primary antibody at the following dilutions: 1:250 for anti-pro-OP-1 antibody R2854 and anti-mature OP-1 antibody, #MAB354, obtained from R&D Systems, and 1:100 for anti-mature OP-1 antibody #SC-9305 obtained from Santa Cruz. Either ImmunoPure Goat Anti-Mouse IgG (Pierce, Rockford, IL) or Donkey Anti-Rabbit IgG (Pierce, Rockford, IL) conjugated with horseradish peroxidase was diluted 1:10,000 and used as the second antibody. The Western blots were developed using an ECL-PLUS kit (Amersham Life Science, England). The specificity of binding of the antibodies to recombinant pro- or mature OP-1 was confirmed according to standard methods. Secondary antibodies were also tested for non-specific binding according to standard methods.

### Chemiluminescent OP-1 Sandwich ELISA

For the sandwich ELISA, two antibodies, one polyclonal #SC-9305 (Santa Cruz) and one monoclonal 1B12 (Stryker Biotech) were used. Polyclonal anti-OP-1 antibody #SC-9305 was used as the plate coating antibody and 1B12 was used as the second antibody. Plates were coated with 50 ng/well #SC-9305 in Tris-buffered saline (TBS), pH 7.5, and incubated overnight at 4°C. Plates were washed four times with TBS/T (0.1% Tween 20 in TBS, pH 7.5) Non-specific binding was blocked by incubation at room temperature (RT) for 2 hours with 20ul/well blocking solution containing 5% non fat dry milk (Bio-Rad #170-6404, Hercules, CA) in TBS/T, pH 7.5. Plates were washed four times with TBS/T.

To generate a standard curve, mature recombinant OP-1 (Stryker Biotech) was diluted in TBS/T to various concentrations ranging from 10 ng/ml to 0.01 ng/ml. 100 µl of either OP-1 solution or a cartilage extract was added to a plate well (in triplicate) and incubated for 1 hour at room temperature. Plates were washed four times with TBS/T. 100 µl of 1B12, diluted 1:1000 in TBS/T, was added to each well and incubated at room temperature for 1 hour. Plates were washed four times with TBS/T. 100 µl of ImmunoPure goat anti-mouse IgG peroxidase-conjugated antibody (Pierce, #31434), diluted 1:10,000 in TBS/T, was then added to each well and incubated plate at RT for 1 hour. Plates were washed four times with TBS/T. The reaction was developed by adding 100 µl Supersignal ELISA Femto Maximum Sensitivity Substrate (Pierce, # 37075) (prepared by mixing equal parts of Supersignal ELISA Femto Luminol/Enhancer solution and Supersignal ELISA Femto Stable peroxide solution) and shaking for 1 minute on a shaker. The data were obtained as Relative Light Units (RLUs) using a chemiluminescent ELISA plate reader Victor² (Wallac).

### Reverse Transcription-Polymerase Chain Reaction (RT-PCR):

Total RNA was extracted directly from cartilage tissue using acid-guanidinium thiocyanate as previously described (Cs-Szabo et al. (1997) Arthrit. Rheum. 40:1037). Oligonucleotide primer pairs specific for OP-1 (Chubinskaya et al. 2000) and glyceraldehyde-3-phosphate dehydrogenase (GAPDH) were synthesized. These primer pairs were designed to yield PCR products of different sizes: 319 base pairs (bp) for GAPDH and 313 for OP-1. OP-1 primers used for nested RT-PCR were: a) a 21-mer, antisense, location 1810-1830, 5'-TTTTCCTTTCGCACAGACACC-3' (SEQ ID. NO:1); b) a 20-mer, sense, location 1328-1347, 5'-TGCCATCTCCGTCCTCTACT-3' (SEQ ID. NO:2); and c) a 23-mer, sense, location 1518-1540, 5'-TTCCCCTCCCTATCCCCAACTTT-3' (SEQ ID. NO:3). The specificity of the primers was shown previously (Chubinskaya et al. 2000). GAPDH primers were: sense primer 5'-GGTATCGTGGAAGGACTCAT-3' (SEQ. ID NO:4) and antisense primer 5'-ACCACCTGGTGCTCAGTGTA-3' (SEQ. ID NO:5). Approximately 1 µg of total RNA was transcribed using reverse transcriptase as described by Cs-Szabo et al. (1997). Five µl of the resulting cDNA was amplified by polymerase chain reaction (PCR) using Taq DNA polymerase (Promega, Madison, WI) in the presence of specific upstream and downstream primers (15 pM each; primers (a) and (b) for OP-1 mRNA). 0.5 µl of the first amplification product and a second sense primer (primer (c); nested primer) were used for a subsequent amplification step. In order to perform RT-PCR at optimal conditions and to stay within the logarithmically linear product formation, thirty cycles were chosen (45 sec at 95°C, 30 sec at 57°C of annealing temperature and 45 sec at 72°C for the primers used), followed by the final extension for 5 min at 72°C. PCR products were separated in 3% Metaphor agarose gels (FMC BioProducts, Rockland, ME) and visualized by ethidium bromide staining. The density of the bands was measured using a Fluor-S MultiImager (Bio-Rad, Hercules, CA) with attached software program Quantity One (Bio-Rad, Hercules, CA). The density of the OP-1 bands was normalized to the density of the GAPDH bands to control variability among samples.

### Statistical Analysis

All results shown are mean ± S.E. of at least three separate experiments, with triplicate determination for each point. Covariation significance was determined by Pearson correlation of normal data.

### Results

### OP-1 Sandwich ELISA

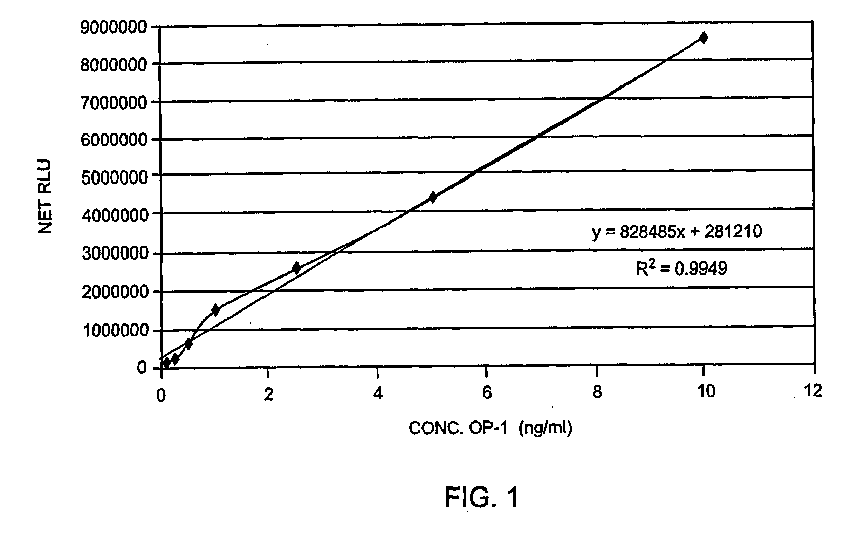

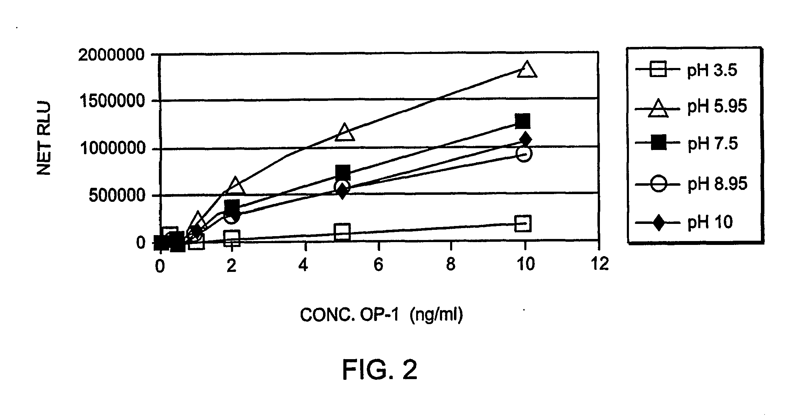

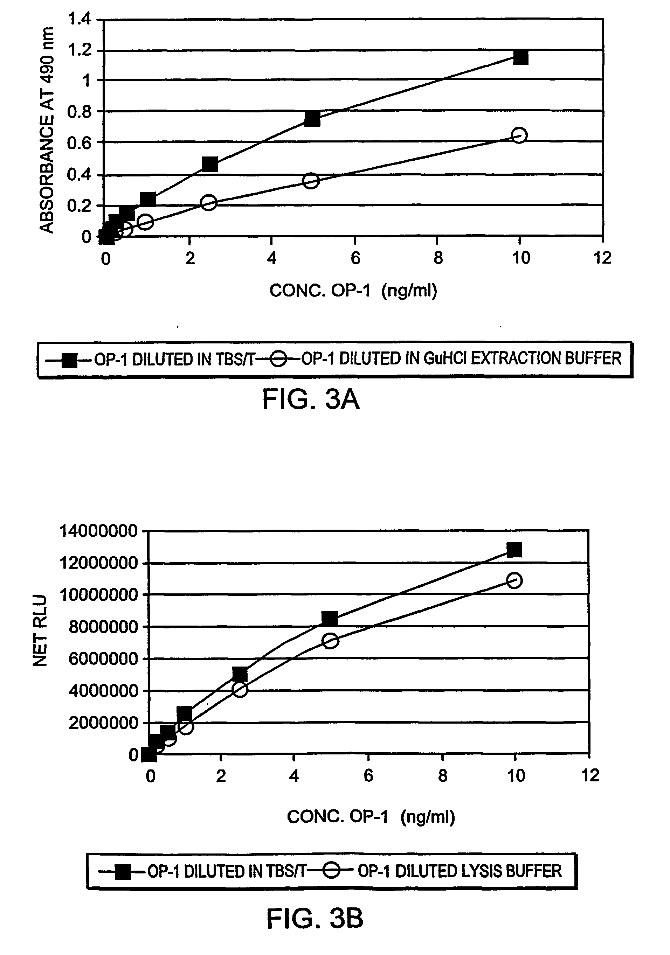

Several parameters were tested in order to optimize the sandwich ELISA for OP-1, including different anti-OP-1 antibody combinations, the effect of pH on the sensitivity of the assay, the effect of various extraction buffers and enzymes, and normalization of OP-1 concentrations to the total protein content or dry weight. The most sensitive assay was that which used the combination of polyclonal #SC-9305 (Santa Cruz) as the coating antibody and monoclonal 1B12 (Stryker Biotech) as the second antibody. FIG. 1 illustrates an exemplary standard curve prepared using human recombinant mature OP-1 at different dilutions ranging from 0.01 to 10 ng/ml. FIG. 2 illustrates the effect of a wide range of pHs for TBS/Tween buffer used in the assay. Since the extraction buffer has a neutral pH and at neutral pH the ELISA readings were in the middle range, this pH was chosen as a standard for the OP-1 Sandwich ELISA. In order to determine whether cartilage extracts have to be dialyzed prior to their assessment using the ELISA two extraction buffers, 1 M GuHCI and lysis buffer, were tested. As shown in FIG. 3A, 1M GuHC1 buffer inhibited the binding of OP-1 by 50% or more, while the lysis buffer did not affect the ELISA results (FIG. 3B). However, when the same cartilage specimens were extracted with both buffers and then analyzed by ELISA, more antigenic OP-1 could be extracted from the cartilage tissue with GuHCl buffer than with lysis buffer. To overcome this problem, the GuHCl buffer was selected as an extraction buffer, but all samples were dialyzed prior to their use in further analyses.

To standardize the ELISA method and address the possible decrease in cell numbers and depletion of matrix molecules that influence cartilage extractability with aging, prior to extraction 500 mg of tissue wet weight is always lyophilized (to avoid the influence of matrix), pulverized in liquid nitrogen and extracted with 1M GuHCl in a ratio of 150 mg (dry weight) of tissue per 3.5 ml of buffer. A repeated extraction of the remaining tissue with 4 M GuHC1 buffer showed no extractable OP-1 left, as detected by ELISA. To confirm quantitatively that 1M GuHCl buffer extracts the most OP-1 protein, aliquots of the same cartilage sample were extracted with a variety of extraction buffers including: 1) 1 M GuHCl, 0.005 M EDTA, 0.05 M NaCl; 2) 50 mM Tris, 1 M GuHCl; 3) 50 mM Tris, 4 M GuHCl; 4) 50 mM Tris, 20 mM Na₂HP0₄; 5) 50 mM Tris, 1% SDS; 6) 50 mM Tris, 0.15 M β-mercaptoethanol; 7) 50 mM Tris, 0.1 M NaCl, 8 M Urea; and 8) 50 mM Tris, 1 M NaCl, 8 M Urea. All these buffers were quantitatively analyzed using the OP-1 sandwich ELISA. The 1M GuHCl buffer was the most appropriate for OP-1 extraction from human adult articular cartilage.

In order to obtain the most sensitive ELISA conditions, different enzymatic treatments of cartilage extracts were tested. Cartilage extracts were treated with proteinase K, hyaluronidase, collagenase, chondroitinase ABC (CHase), keratanase (Ker), or keratanase II (KerII), or combinations of these enzymes, and analyzed by the OP-1 sandwich ELISA. The ELISA values of the treatment groups were compared to those obtained with no prior treatment (Table 1). There was no significant differences between the groups. Therefore, enzymatic digestion is not included in the standard protocol for ELISA.

**Table 1. Comparison of ELISA values with different enzymatic digestions.**

| **TYPE OF TREATMENT** | **ELISA (ng/ml)** |
|---|---|
| 1 M GuHCl buffer only | 0.03 ± 0.0027 |
| 1 M GuHCl buffer + CHase, Ker, Ker II | 0.027 ± 0.0019 |
| 1M GuHCl buffer + CHase, Collagenase | 0.022 ± 0.0024 |
| 1M GuHCl buffer + Hyaluronidase | 0.025 ± 0.0027 |
| 1M GuHCl buffer + Collagenase | 0.0181 ± 0.0011 |
| Lysis buffer | 0.017 ± 0.0013 |
| Lysis buffer + CHase, Collagenase | 0.017 ± 0.0009 |

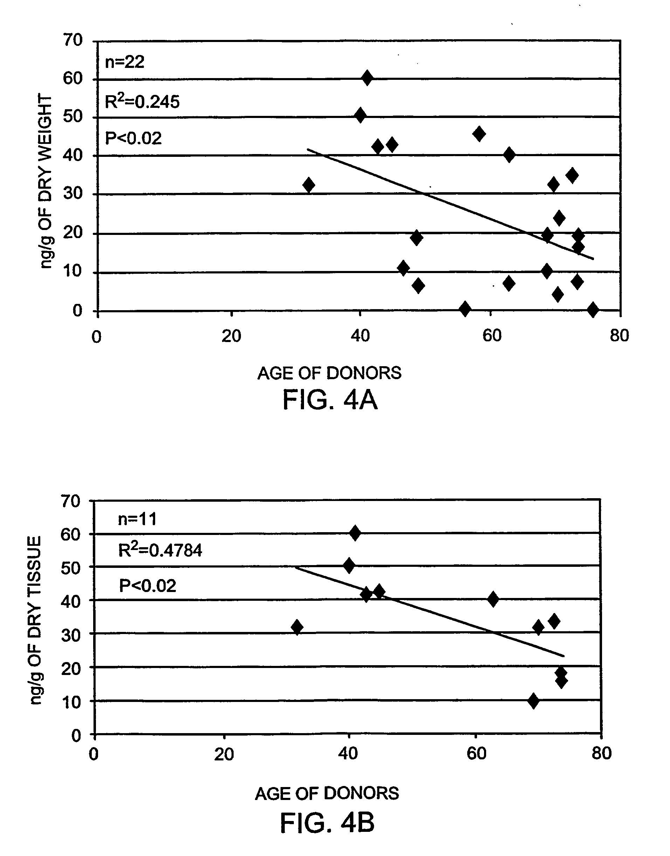

Extracts from all cartilage samples from normal patients of various ages (Collins grade 0-2) were analyzed with OP-1 sandwich ELISA described above. Referring to FIG. 4A, the content of endogenous OP-1 protein significantly decreased with increased age (p<0.02). The age of the donors and the levels of OP-1 protein showed significant covariation (Pearson correlation p<0.02). Importantly, among the samples were cartilage samples with normal histomorphological appearance and cartilage samples with degenerative morphological changes, although all of them were obtained from organ donors with no history of joint disease. Referring to FIG. 4B, when the cartilage samples with normal histomorphology were isolated into a separate subgroup and analyzed for the content of OP-1 protein, the same statistical differences were detected. Even with aging of normal cartilage there was a decrease in the content of endogenous OP-1. In adult tissues, the changes in OP-1 protein expression had a linear regression. There was at least a 3-4 fold difference in OP-1 mRNA levels between the ages of 40 and 70.

### Western Blot Analysis

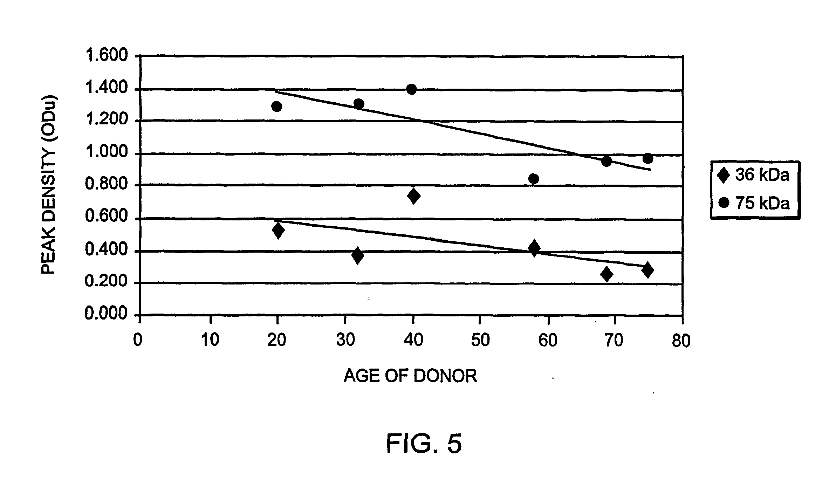

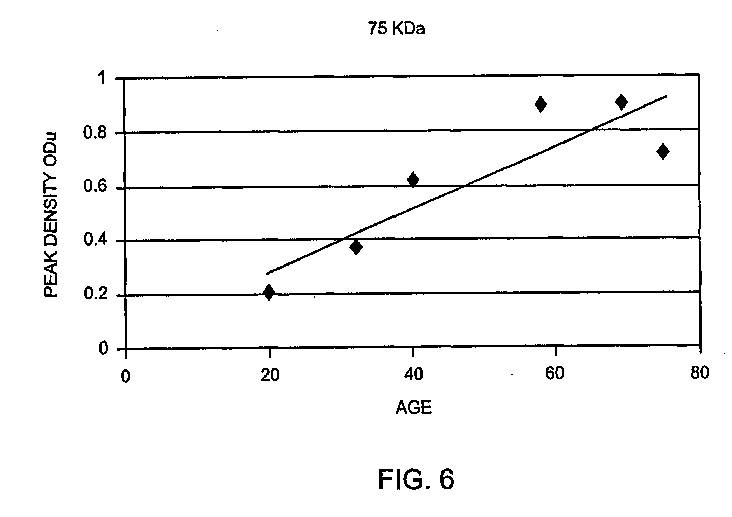

Representative samples were taken from each age decade (20, 32, 40, 58, 69 and 75 years old) and analyzed by Western Blot using an anti-mature OP-1 antibody #MAB354. The gels were scanned and densitometry was performed according to standard methods. Two major OP-1 bands were present in all tested cartilages regardless of the age of donors (data not shown). The bands represented fully processed mature OP-1 (molecular weight about 36 kD) and partially processed intermediate form of the OP-1 protein (molecular weight about 75 kD). Semi-quantitative densitometric analysis of each of these bands demonstrated a statistical decrease in the intensity of these bands with increased age, correlating with the ELISA data (P<0.05). The amount of active mature OP-1 was significantly decreased with increased age. Bands that represent a hemidimer form of OP-1 at 75kD and the mature OP-1 (at 36 and 17kD) disappeared in degenerated and aged cartilage suggesting that the major changes may occur on the levels of processed mature OP-1. FIG. 5 represents a quantitative image analysis of Western Blot bands that correspond to the processed mature OP-1 dimer (36 kD) and the hemidimer form of OP-1 (75 kD). Referring to FIG. 6, when the same cartilage extracts were analyzed with anti-pro-OP-1 antibody R2854 and quantified using an Image analyzer, an increase in the band that corresponds to the hemidimer form of pro-OP-1 protein was observed. Bands at the lower molecular weight (pro-OP-1 reduced monomer and pro-OP-1 domain) disappeared with cartilage aging.

### RT-PCR.

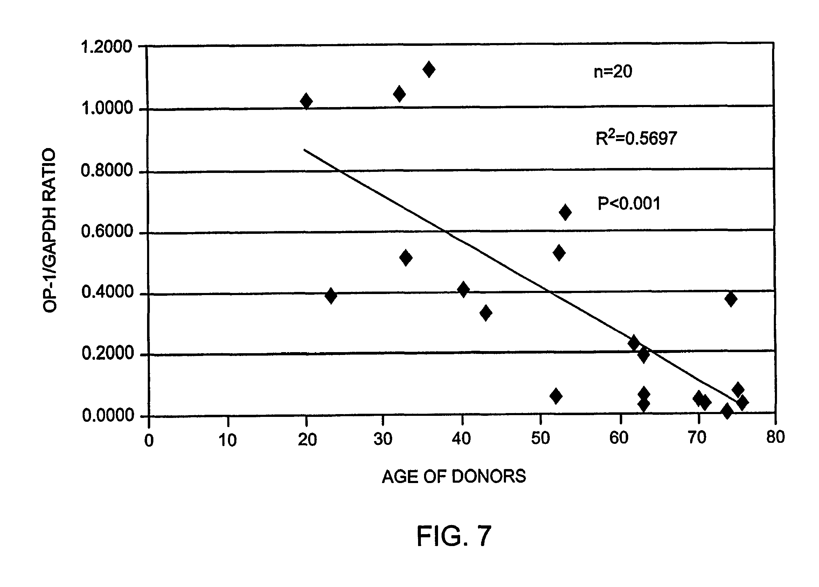

Total RNA was extracted directly from cartilage tissue without chondrocyte isolation or culture and subjected to nested RT-PCR analysis using OP-1 and GAPDH specific primer sets. Both PCR products were amplified for the same number of cycles. GAPDH was chosen as a normalization factor because it is a housekeeping gene and because levels of GAPDH mRNA expression in normal cartilage do not vary by more than 10% with age. FIG. 7 illustrates OP-1/GADPH ratios plotted versus the age of cartilage donors. OP-1 mRNA levels in articular cartilage decreased with increasing age of donors (P<0.001). The highest levels of OP-1 mRNA were detected in newborn and young adult donors, while OP-1 expression was markedly down regulated throughout the aging process. In adult tissues, the changes in OP-1 mRNA levels had a linear regression. There was at least a 4-5 fold difference in OP-1 mRNA levels between the ages of 30 and 80. By age 80, OP-1 mRNA levels were very low or barely detectable, with some donors having OP-1 mRNA levels below the detection limit.

### Example 2: OP-1 Protein and mRNA levels in Rheumatoid Arthritis and Osteoarthritis

The above-described OP-1 sandwich ELISA was used to determine whether OP-1 protein could be detected in synovial fluid, whether quantitative approaches could be adapted for the assessment of OP-1 protein in synovial fluid, and whether there are differences in the levels of OP-1 protein between normal donors and patients with rheumatoid arthritis (RA) and osteoarthritis (OA). The results suggest that synovial fluid OP-1 is a useful diagnostic and prognostic marker for both RA and OA.

Synovial fluid was aspirated from subjects with RA and OA as well as from normal joints of human organ donors according to standard methods. Cartilage specimens from 74 joints (13 normal, 25 RA, 29 OA and 7 other inflammatory diseases) were also obtained. Synovial fluid and cartilage was analyzed by Western Blot with anti-pro and anti-mature OP-1 antibodies and the concentration of OP-1 protein was measured using the OP-1 sandwich ELISA, as described in Example 1. The concentration of OP-1 in the samples was quantified in a set of five and tested for at least three to five 5 times. Synovial fluid was diluted 1:100 prior to Western Blot analysis and ELISA.

### Results

### OP-1 Sandwich ELISA Results and RT-PCR

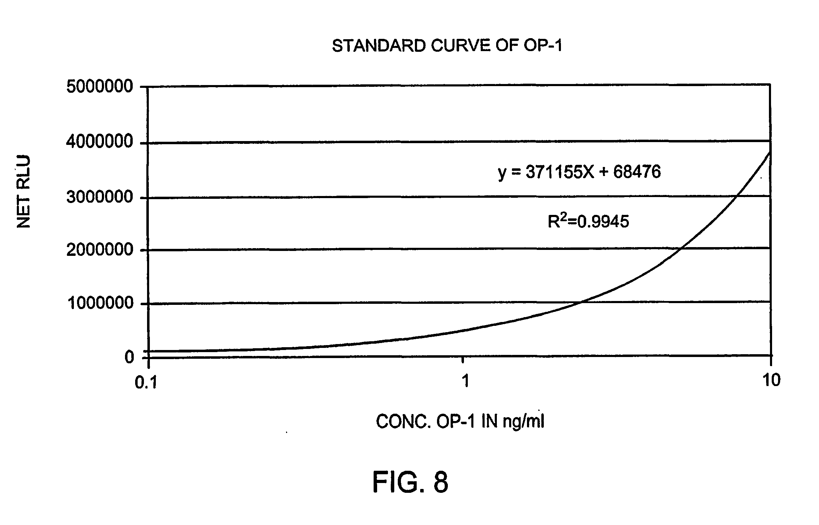

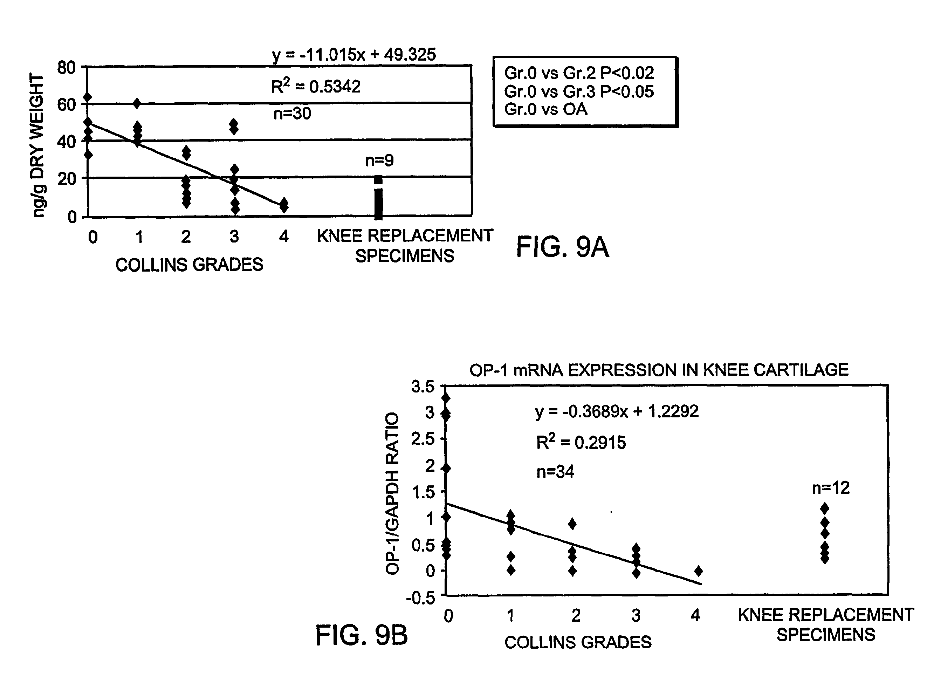

FIG. 8 illustrates a typical standard curve for the ELISA. Pro- and mature forms of OP-1 protein were present in all tested samples. FIG. 9A illustrates the relationship between OP-1 protein concentration in cartilage and the progression of cartilage degradation in normal and OA patients. An increase in Collins grade or the presence of OA in a patient correlated with a significant decrease in endogenous OP-1 protein concentration. The levels of endogenous OP-1 protein in cartilage of grades 2 and 3 were two-fold decreased when compared to cartilage of grades 0 and 1 (p<0.02 and p<0.05, respectively), and four to six-fold decreased in cartilage of grade 4 and from OA patients (p<0.003).

The same cartilage samples analyzed by ELISA in FIG. 9A were examined for OP-1 mRNA concentration with RT-PCR. Referring to FIG. 9B, OP-1 mRNA expression was two-fold decreased in cartilage of grades 2 and 3 when compared to cartilage of grades 0 and 1 (p<0.05). However, in OA cartilage the levels of OP-1 mRNA were comparable to the levels in normal tissue (grades 0 and 1). These results suggest that OA tissue elicits an anabolic response for regeneration of cartilage tissue. Notably, the concentration of OP-1 protein in synovial fluid from organ donors was comparable to that detected in cartilage extracts from the same donors. OP-1 protein concentration was higher in donors with normal knee joints than in donors with degenerative changes (p<0.015). OP-1 protein concentration was higher in synovial fluid obtained from RA patents than in that obtained from OA patents (p<0.03).

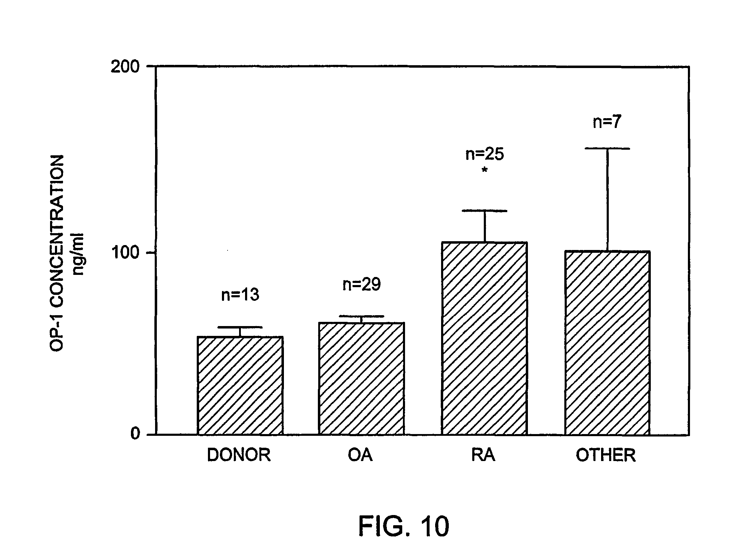

FIG. 10 illustrates the level of OP-1 protein in human synovial fluid from normal, OA, and RA patients, as well as other patients with various other types of arthritis, such as gout, fibromyalgea syndrome (FMS), and polymyalgea rheumatica (PMR). OP-1 protein in synovial fluid is increased in patients with OA and RA and the concentration of OP-1 protein is at least two-fold higher in synovial fluid from RA patients and the group that combined all other types of arthritis compared to normal or OA patients.

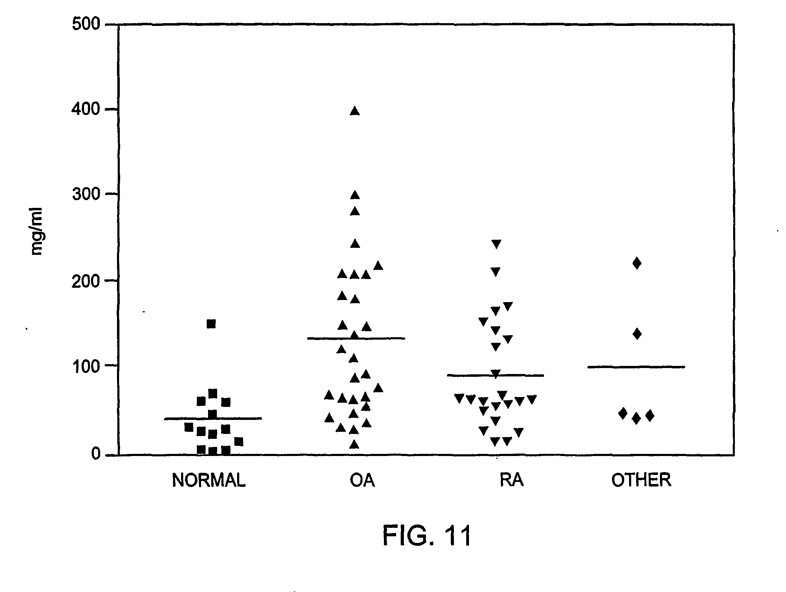

However, when these increases take into account the total protein present in synovial fluid, the results show a marked decrease in OP-1 protein in OA and RA patients. FIG. 11 illustrates the amount of total protein in synovial fluid from patients with OA, RA, and other diseases. Total protein is increased in all diseased tissues.

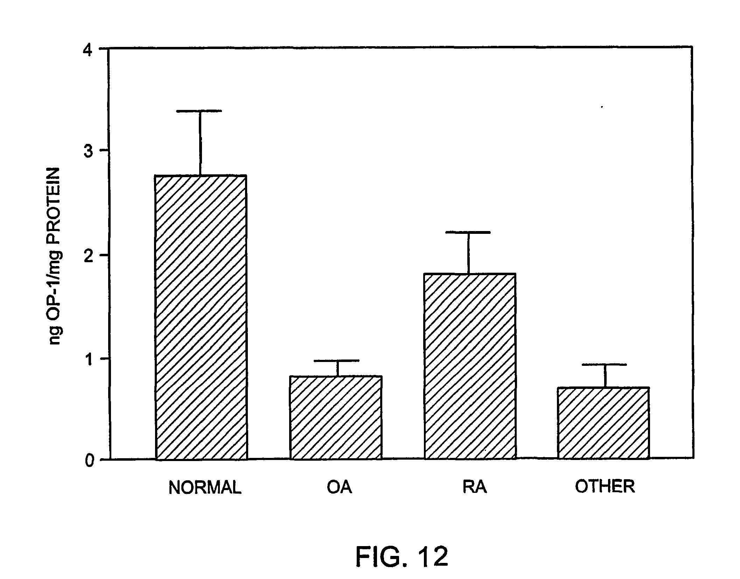

Referring to FIG. 12, when the OP-1 protein concentrations in synovial fluid are normalized to total protein concentration, OP-1 protein is decreased dramatically in OA patients. OP-1 protein is also decreased in RA patients, but to a lesser extent than in OA patients; the normalized concentration of OP-1 protein is also two-fold higher in synovial fluid from RA patients.

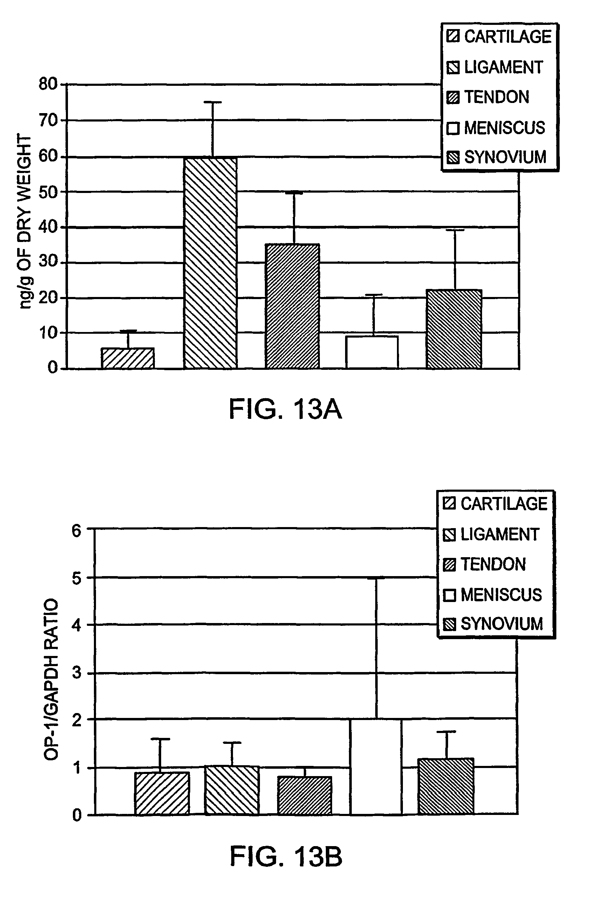

OP-1 protein and mRNA expression was examined in other connective tissues from the knee joint. Ligament, tendon, meniscus and synovium were obtained from the knee joint of normal human donors with a Collins grade of 2. ELISA and RT-PCR were performed as described in Example 1. Referring to FIG. 13A, OP-1 protein was detected in all tissues, with the greatest levels of OP-1 protein detected in ligament, tendon and synovium. Referring to FIG. 13B, OP-1 mRNA was detected in all tissues at similar levels, with higher levels detected in the tendon.

### Western Blot

Western Blot analyses demonstrated that the distribution of immunoreactive bands of OP-1 from synovial fluid was similar to that described for human articular cartilage (not shown). Western Blot analyses of synovial fluid digested with hyaluronidase and/or chondroitinase showed that these enzymes did not alter the pattern of immune bands detected by anti OP-1 antibody. Based on these results, synovial fluid was not subjected to enzymatic digestion prior to ELISA.

### Example 3: OP-1 protein and mRNA Levels Decrease in Osteoarthritis

Human normal cartilage derived from normal newborn and normal adult donors with no documented history of joint disease were obtained according to standard procedures. Osteoarthritis cartilages (OA) were removed from patients diagnosed with OA who underwent knee arthroplasty. Three samples of each type were tested. RT-PCR of OP-1 and GADPH mRNA was performed as described in Example 1. Levels of OP-1 mRNA in normal newborn and normal adult cartilage were similar, whereas OP-1 mRNA expression in OA cartilage was up-regulated two to three-fold (Table 2).

**Table 2: OP-1 mRNA Expression In Human Articular Cartilage**

| **Type of Cartilage** | **OP-1/GADPH** | **# specimens** |
|---|---|---|
| Normal newborn cartilage | 0.518 ± 0.066 | N=3 |
| Normal adult cartilage | 0.567 ± 0.067 | N=3 |
| OA cartilage | 1.148 ± 0.234 | N=3 |
| | P<0.01 | |

OP-1 protein was extracted from tissues with 1M GuHCl in the presence of protease inhibitors, lyophilized, and analyzed by Western Blot under non-reduced conditions, as described in Example 1. The density of immunoreactive OP-1 protein bands was quantified with Quantify One Software attached to a Fluor-S MultiImager (BioRad). All data was normalized to the total protein content. The total densities of the pro- and mature OP-1 protein were statistically higher in the normal cartilage than in OA cartilage, indicating the higher content of pro- and mature OP-1 protein in normal tissue when compared to OA tissue (Table 3). The results suggest that OA chondrocytes compensate for the process of tissue degeneration by an up-regulation of OP-1 mRNA expression but which is not apparent at the protein level.

**Table 3. Total content of pro- and mature OP-1 protein in human articular cartilage**

| **Type of cartilage** | **Pro-OP-1** | **Mature OP-1** | **# of specimens** |
|---|---|---|---|
| Normal cartilage | 0.69 ± 0.26 | 1.05 ± 0.19 | N=4 |
| OA cartilage | 0.37 ± 0.05 | 0.41 ± 0.10 | N=4 |
| | P<0.05 | P<0.001 | |

### Example 4: Regulation of OP-1 Expression In Vitro Using IL-1β

The response of the OP-1 gene to treatment with IL-1β, a catabolic mediator known to be associated with cartilage destruction *in vitro,* was examined. IL-1 β was chosen as a catabolic model of the initial changes in human articular cartilage during inflammation.

Normal cartilage was obtained from femoral condyles of the knee joints from human organ donors with no documented history of joint disease. Cartilage slices were prepared and briefly washed in Dulbecco's Modified Eagle's Medium (DMEM), cut into 3-5mm square explants and cultured under standard culture conditions: 50% DMEM, 50% Ham's F12, supplemented with 25µg/ml ascorbic acid, 50µg/ml gentamicin and 10ml/L of Insulin-Transferrin-Selenium A (ITS, Gibco). ITS was chosen to reduce the effect of growth factors present in FBS on endogenous OP-1 expression. Cartilage explants were incubated at 37°C with 7% CO₂ in a humidified atmosphere with changes of medium every other day. IL-1β was added to explant cultures at a low dose of 0.1ng/ml IL-1β or a high dose of 1.0 ng/ml IL-1β (six donors each). Tissue slices were given 4 days to adjust to standard culture conditions (OP-1 near steady state) prior to IL-1β treatment; then explants were treated by IL-1β for 48 or 96 hours. Media was changed and collected every other day. After culture, explants were processed for protein extraction and analyzed for the content of OP-1 protein by Western Blot and ELISA. The same tissue extracts were utilized to detect PG levels (content of sulfated glycosaminoglycans (GAGs) by the standard DMMB method. Culture media was assayed for GAG and OP-1 release. Normal cartilage from 3 additional donors was cultured under identical conditions as a control and treated with a low dose and high dose of IL-1β. This tissue was collected and processed for RNA extraction.

### Response to Low Dose of IL-1β

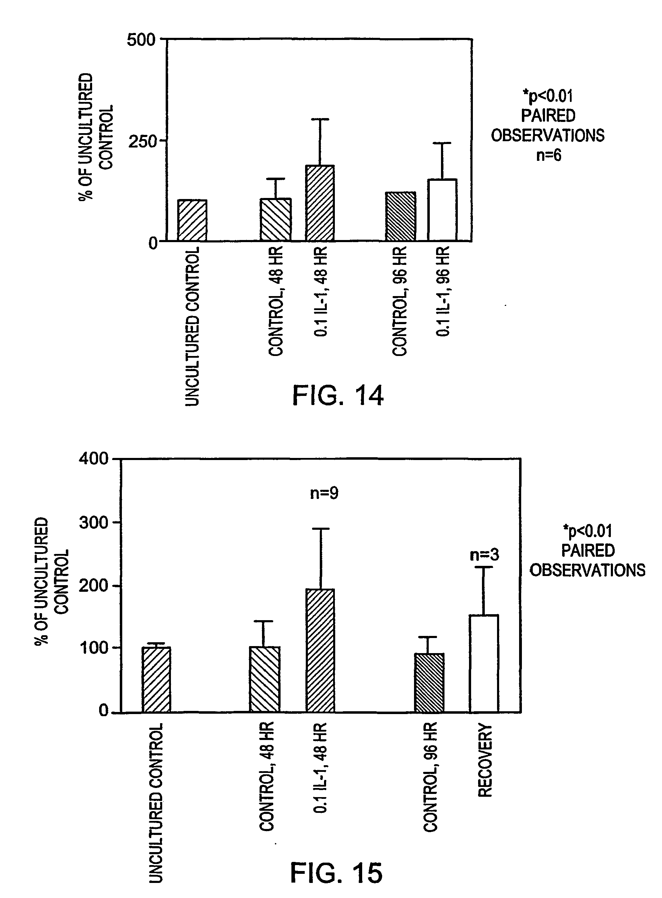

Cartilage from 6 normal donors was used to examine the response of OP-1 to a low dose of IL-1β. After a 4 day equilibration period (culture in the presence of media only), explants were treated with 0.1ng/ml of IL-1β for 48 or 96 hours. Referring to Figure 14, culture for 48 hours in the presence of a low IL-1β dose led to significant accumulation of endogenous OP-1 protein (p<0.01); however longer exposure to IL-1β (96 hours) induced a lesser increase in endogenous OP-1 protein. In 5 out of 6 cartilage extracts, culture of 48 hours with a low dose of IL-1β caused a 2-3 fold increase in the concentration of endogenous OP-1 over cultured controls. After 96 hours OP-1 protein levels were 1.5 times higher in IL-1β treated groups (p<0.01). These results suggest an overall increase in the levels of endogenous OP-1 in response to treatment with a low dose of IL-1β.

ELISA analysis of cultured media for the content of released OP-1 showed no detectable levels of OP-1 protein in the media. This suggests either a rapid degradation of the released OP-1 or that the concentration of OP-1 protein in the media is below the detection limit of the assay.

After the finding that longer exposure to IL-1β (96 hours) does not lead to further induction of OP-1 protein in comparison to a shorter culture (48 hours), the changes in endogenous OP-1 protein levels after removal of IL-1β was tested. Cartilage samples were cultured first for 48 hours in the presence of 0.1ng/ml IL-1β, the IL-1β was removed and cartilage was cultured for an additional 48 hours in media only. Referring to FIG. 15, the ELISA analysis indicated that after the removal of IL-1β, the levels of endogenous OP-1 remained elevated for at least another 48 hours, although the absolute values after the recovery were lower than in the presence of IL-1β (both 48 and 96 hours)

The appearance of OP-1 immunoreactive bands was analyzed by Western Blot. In cartilage extracts treated with a low dose of IL-1β, three major bands were detected: bands that correspond to mature OP-1 dimer, intermediate forms of OP-1 and monomers or degradation fragments. After both 48 and 96 hours of treatment with low dose IL-1β, the intensity of the band that corresponds to mature OP-1 was stronger in IL-1β treated extracts than in untreated controls. No differences were detected in the OP-1 bands that correspond to the intermediate forms of OP-1. Media collected from IL-1β treated cultures was also analyzed by Western Blot and no OP-1 was detected.

Levels of OP-1 mRNA were examined to determine if the IL-1β induced elevation in OP-1 protein was related to changes in its mRNA expression. Cartilage samples from three separate donors were cultured under the same conditions and OP-1 mRNA levels determined. Results were reported as ratios of OP-1 mRNA to GADPH mRNA. The same number of cycles was used to generate both gene products. After 48 hours of treatment with a low dose of IL-1β, there was no statistical difference in OP-1 mRNA expression between IL-1β treated and untreated (control) tissue samples. However, at 96 hours in the presence of IL-1β, OP-1 mRNA was 30% higher.

### Response to High Dose of IL-1β

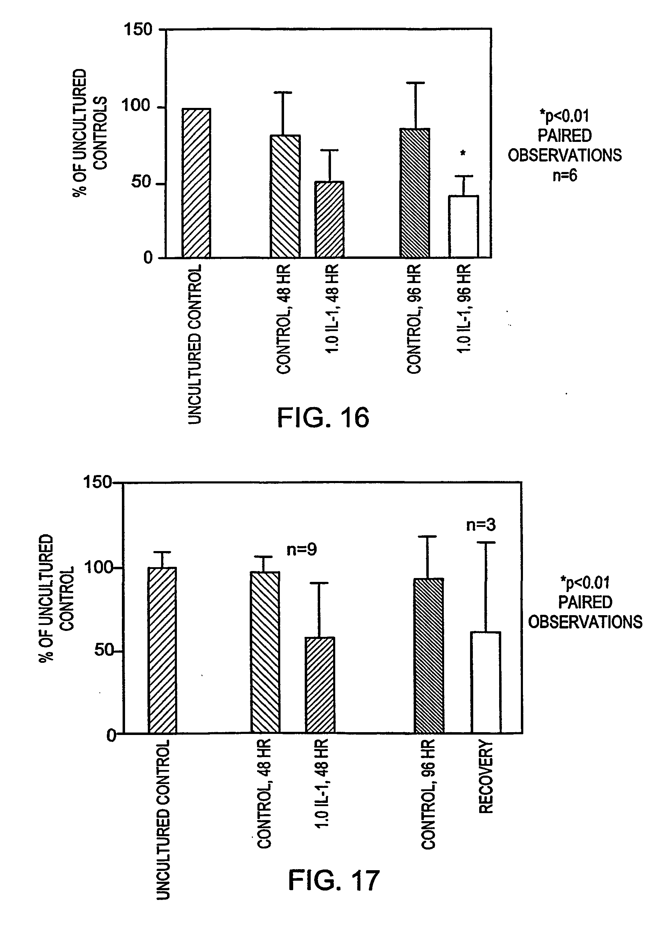

Cartilage samples were obtained as described above for the low dose experiments and were treated instead with a high dose (1.0ng/ml) of IL-1β and analyzed by ELISA. Referring to FIG. 16, after 48 hours and 96 hours treatment with a high dose of IL-1β, extracts from each of the six cartilage samples showed a similar decrease in the levels of OP-1 protein. Referring to FIG. 17, after removal of the IL-1β and 48 hours of recovery in culture the level of endogenous OP-1 protein did not reach the levels detected in the culture control.

Levels of OP-1 mRNA were measured by RT-PCR (as described in Example 1) in response to a high dose of IL-1β. After 48 hours of culture, there was an increase in OP-1 mRNA concentration (about 40%, p<0.05).

## Claims

1. An in-vitro method of determining either: i) a predisposition for rheumatoid arthritis or osteoarthritis; or ii) the presence of rheumatoid arthritis or osteoarthritis in a patient, the method comprising the steps of:
(a) determining a normalized amount of i) OP-1 protein or ii) OP-1 mRNA that is present in a synovial fluid sample from the patient; and
(b) comparing said normalized amount of i) OP-1 protein or ii) OP-1 mRNA in a predetermined standard;
wherein a difference in the normalized amount of i) OP-1 protein or ii) OP-1 mRNA present in said sample and the predetermined standard is indicative of either: i) a predisposition for rheumatoid arthritis or osteoarthritis, or ii) the presence of rheumatoid arthritis or osteoarthritis.

2. The method of claim 1, wherein the predetermined standard comprises a range of values.

3. A method according to claim 1, wherein said predetermined standard is correlated with the age of said patient and is representative of a normalized amount of OP-1 protein expected to be present in a clinically-normal joint region.

4. A method claimed in claim 1 of determining the clinical status of a joint region of a patient, wherein the predetermined standard is indicative of a normalized amount of OP-1 protein expected to be present in a clinically normal joint region; and wherein an amount determined in step a) that is about equal to said standard is indicative of a normal clinical status of said joint region of said patient and an amount that is not about equal to said standard is indicative of an abnormal clinical status of said joint region of said patient.

## Patentansprüche

1. In-vitro-Verfahren zur Bestimmung entweder: i) einer Prädisposition für rheumatoide Arthritis oder Osteoarthritis, oder ii) der Gegenwart von rheumatoider Arthritis oder Osteoarthritis bei einem Patienten, wobei das Verfahren die Schritte aufweist:
(a) Bestimmen einer normalisierten Menge von i) OP-1-Protein oder ii) OP-1-mRNA, die in einer Synovialflüssigkeitsprobe von dem Patienten vorhanden ist, und
(b)Vergleichen der normalisierten Menge von i) OP-1-Protein oder ii) OP-1-mRNA mit einem vorbestimmten Standard,
wobei ein Unterschied in der normalisierten Menge von i) OP-1-Protein oder ii) OP-1-mRNA, der in der Probe und dem vorherbestimmten Standard vorhanden ist, für entweder: i) eine Prädisposition für rheumatoide Arthritis oder Osteoarthritis oder ii) die Gegenwart von rheumatoider Arthritis oder Osteoarthritis anzeigen kann.

2. Verfahren nach Anspruch 1, wobei der vorbestimmte Standard einen Bereich von Werten aufweist.

3. Verfahren gemäß Anspruch 1, wobei der vorbestimmte Standard mit dem Alter des Patienten korreliert ist und repräsentativ für eine normalisierte Menge OP-1-Protein ist, die in einem klinisch normalen Gelenkbereich als vorhanden erwartet wird.

4. Verfahren wie in Anspruch 1 beansprucht, zur Bestimmung des klinischen Status eines Gelenkbereiches eines Patienten, wobei der vorherbestimmte Standard indikativ für eine normalisierte Menge von OP-1-Protein ist, die in einem klinisch normalen Gelenkbereich als vorhanden erwartet wird, und wobei eine in Schritt a) bestimmte Menge, die etwa gleich zu dem Standard ist, indikativ für einen normalen klinischen Status des Gelenkbereiches des Patienten ist, und eine Menge, die nicht etwa gleich zu dem Standard ist, indikativ für einen abnormalen klinischen Status des Gelenkbereiches des Patienten ist.

## Revendications

1. Procédé in vitro pour déterminer : i) une prédisposition à l'arthrite rhumatoïde ou ostéoarthrite, ou ii) la présence d'arthrite rhumatoïde ou ostéoarthrite chez un patient, le procédé comportant les étapes consistant à :
(a) déterminer une quantité normalisée de i) protéine OP-1 ou ii) d'ARNm de OP-1 qui se trouve dans un échantillon de fluide synovial provenant du patient, et
(b) comparer ladite quantité normalisée de i) protéine OP-1 ou ii) d'ARNm de OP-1 avec une norme prédéterminée,
dans lequel une différence entre la quantité normalisée de i) protéine OP-1 ou ii) d'ARNm de OP-1 se trouvant dans ledit échantillon et la norme prédéterminée indique : ii) une prédisposition à l'arthrite rhumatoïde ou ostéoarthrite, ou ii) la présence d'arthrite rhumatoïde ou ostéoarthrite.

2. Procédé selon la revendication 1, dans lequel la norme prédéterminée comporte une plage de valeurs.

3. Procédé selon la revendication 1, dans lequel ladite norme prédéterminée est mise en corrélation avec l'âge dudit patient, et est représentative d'une quantité normalisée de protéine OP-1 censée être présente dans une zone d'articulation normale cliniquement.

4. Procédé selon la revendication 1, consistant à déterminer l'état clinique d'une zone d'articulation d'un patient, dans lequel la norme prédéterminée indique une quantité normalisée de protéine OP-1 censée être présente dans une zone d'articulation normale cliniquement, et dans lequel une quantité déterminée à l'étape a) qui est environ égale à ladite norme indique un état clinique normal de ladite zone d'articulation dudit patient, et une quantité qui n'est pas environ égale à ladite norme indique un état clinique anormal de ladite zone d'articulation dudit patient.
